# EUROPEAN PATENT SPECIFICATION

(11) **EP 2 053 049 B1**
(45) Date of publication and mention of the grant of the patent: **21.09.2011**
(21) Application number: 07020873.1
(22) Date of filing: 25.10.2007
(51) Int. Cl.: C07D 401/14

(54) **Preparation of 2' , 6' -di-pyrazolyl-pyridine containing a reactive group R in 4' position**
Herstellung von 2',6'-Dipyrazolylpyridin mit einer reaktiven R-Gruppe in Position 4
Préparation de 2' , 6' -di-pyrazolyl-pyridine contenant un groupe réactif R en position 4'

(43) Date of publication of application: 29.04.2009
(73) Proprietor: Karlsruher Institut für Technologie, 76131 Karlsruhe (DE)
(72) Inventor: Rajadurai, Chandrasekar, Athikulam, Madurai-625007, Tamil Nadu (IN); Ruben, Mario, 6700 Strasbourg (FR)
(74) Representative: Gärtner, Stephan

(56) References cited:
- RAJADURAI, CHANDRASEKAR ET AL: "Spin Transition in a Chainlike Supramolecular Iron(II) Complex" INORGANIC CHEMISTRY, vol. 45, no. 25, 2006, pages 10019-10021, XP002472201
- ELHAIK ET AL: "Synthesis of 2,6-di(pyrazol-1-yl)-4-bromomethylpyridine , and its conversion to other 2,6-di(pyrazol-1-yl)pyridines substituted at the pyridine ring" TETRAHEDRON, ELSEVIER SCIENCE PUBLISHERS, AMSTERDAM, NL, vol. 63, no. 2, 5 December 2006 (2006-12-05), pages 291-298, XP005802252 ISSN: 0040-4020

## Description

The present invention relates to the preparation of 2',6'-dipyrazolyl-pyridine containing a reactive amino group R in 4' position

Tridendate ligands are often used as ligands in the field of coordination chemistry, for instance in bio-inorganic, molecular magnets or spin transition complexes.

2',6'-di-pyrazolyl-pyridine and its use as a ligand in coordination chemistry is known from the documents C. Rajadurai, O. Fuhr, R. Kruk, M. Ghafari, H. Hahn and M. Ruben, Chem. Commun., 2007, 2636-2638, C. Rajadurai, F. Schramm, S. Brink, O. Fuhr, M. Ghafari, R. Kruk and M. Ruben, Inorganic Chemistry, Vol. 45, No. 25, 2006, 10019-10021, C. Rajadurai, Z. Qu, O. Fuhr, B. Gopalan, R. Kruk, M. Ghafari and M. Ruben, Dalton Trans., 2007, 3531-3537.

These documents also teach a method of preparing the ligand 2',6'-di-pyrazolyl-pyridine and its derivates. However, this method seems to be too complicated. It consists of five separate steps. The overall yield of these consecutive steps is very poor and the used chemicals are rather expensive. The reaction time is long and the following work-up procedure is difficult. Therefore an alternative method for the preparation of 2',6'-di-pyrazolyl-pyridine, especially for its derivates containing a reactive group R in 4' position, is needed.

It is an object of the invention to propose a improved and less complicated method of preparation of 2',6'-di-pyrazolyl-pyridine containing a reactive amino group R in 4' position with fewer separate steps, cheaper chemicals and a reduced reaction time.

The solution of this problem is described by the preparation according to claim 1. A preferred method of preparation is described by the dependent claims.

According to the invention, 2',6'-di-pyrazolyl-pyridine containing a reactive amino group R in 4 position reacts with an alkali metal salt of pyrazole. The 2',6'-di-pyrazolyl-pyridine containing a reactive group in 4 position contains chlorine atoms in 2- and 6-position.

As alkali metal salts of pyrazole potassium salts are preferred, but lithium, sodium, rubidium and caesium salts can be used in the same way. The solvent for the reaction is preferably an ether with an boiling point above 100 °C, preferably a polyether, most preferably diglyme.

The reactive amino group in 4 position of the pyrazole ring in 2',6'-di-pyrazolyl-pyridine is important to react the molecule with further molecules to prepare new classes of compounds. Examples of such compounds are given below.

The amino derivate of 2',6'-di-pyrazolyl-pyridine can be prepared by reaction of 4-amino-2,6-dichloro-pyrazole with the potassium salt of pyrazole or the pyrazolate anion. As generally known the pyrazolate anion may be prepared by reaction of potassium metal with pyrazole in an ether, preferably in diglyme. By this reaction 4'-amino-2',6'-di-pyrazolyl-pyridine (II) is produced. The reaction conditions are 110 °C / 7 days. The 4'-amino-2',6'-di-pyrazolyl-pyridine may react to the 4'-iodine at 75°C in CH₂Cl₂ as a solvent in the presence of KI₂ and I₂.

The reaction of organometallic compounds with halogen-, especially chlorine substituted organic compounds to connect both organic molecules is well known in the art. However, it is clear that except of the halogen group there are no additional reactive groups allowed in the substituted compound, as these additional reactive groups would react with the organometallic compounds as well. It is evident that this reactive group would not continue to exist in the assembled molecule. Otherwise, it would be necessary to protect the reactive group in complicated additional steps by molecules, which would prevent the attack of the organometallic compound. These molecules must be removed after the synthesis. It is surprising that this possible side reaction does not take place in the preparation according to the invention.

Therefore, the expression "reactive group" means a chemical group, which may react with an organometallic compound.

In the following the synthesis of 4'-amino-2',6'-di-pyrazolyl-pyridine will be described.

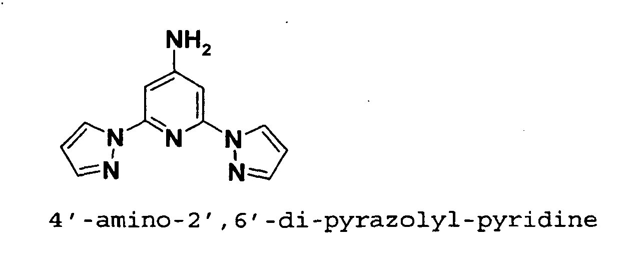

The inventors have developed a synthetic protocol from a commercially available 2,6-dichloro-4-aminopyridine. Transformation of 2,6-dichloro-4-aminopyridine into 4'-amino-2',6'-dipyrazolyl-pyridine requires K metal, pyrazole, an ether, preferably diglyme as a solvent and available 2,6-dichloro-4-aminopyridine. The reaction was effected in a step-wise manner.
a) treating pyrazole to a dispersion containing K metal flakes in diglyme or Bis (2-methoxyethyl) ether solvent under nitrogen/argon atmolsphere at 70 °C for 4 hours with constant stirring to obtain a white turbid solution of pyrazolate anion. 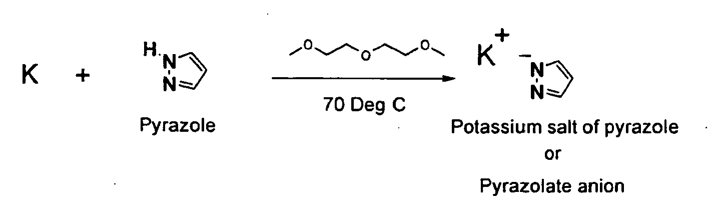
b) reacting the white turbid solution of potassion pyrazolate obtained in step a) with available 2,6-dichloro-4-aminopyridine at 110 °C under nitrogen or argon atmosphere with stirring for 7 days, 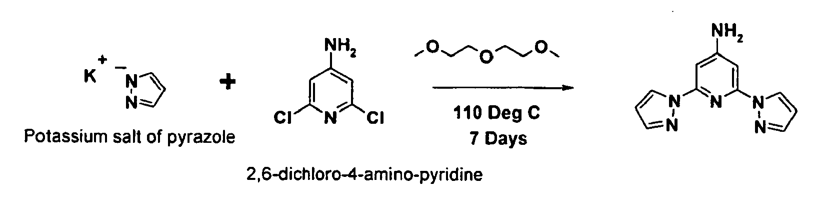
c) pouring the reaction into 500 ml cold water and filtering the formed brown colour precipitate of 4'-amino-2',6'-di-pyrazolyl-pyridine compound.

Although normally amino compounds are very sensitive and reactive to alkali metal, especially to K metal, the amino group remains unreacted according to the invention. This could be achieved in the following way: The K metal (1 mol) was cut into small flakes and dispersed into a high boiling diglyme solvent. Then a slight stiochiometric excess of pyrazole (1.1 mole) was added into it to completely consume al the K metal by forming a white colour potassium salt of pyrazole. This way one can completely eliminate the possibility of the reaction of K with amino group of the 4-amino-2,6-dichloropyridine. The solvent preferred for the reaction is diglyme or (Bis(2-methoxyethyl)ether. Diglyme has the ability to chelate small cations, leaving anions more active. Therefore, reactions involving organometallic reagents, such as Grignard reactions, or metal hydride reductions may have significantly enhanced reaction rates. It also provides high boiling point necessary for the reaction (110 °C) and also stable at higher temperature.

In those above reaction steps K metal reacts with pyrazole by forming a nucleophilic salt i. e. a potassium salt of parazole or a pyrazolate anion. The use of diglyme as a solvent makes the pyrazolate anion mor active due to the formation of chelation complexes of K. And also consumption of al the K metal by taking excess of pyrazole in the step a) facilitate direct nucleophilic reaction of the pyrazolate anion by displacing the halides in the 2,6-position of the 2,6-dichloro-4-aminopyridine without affection the potassium sensitive amino group. The excess unreacted pyrazole can be easily removed during the work-up due to its water solubility. During the work-up the whole reaction mixture was poured into a 500 ml cold water, the water insoluble 4'-amino-2',6'-di-pyrazolyl-pyridine forms a precipitate and the unreacted pyrazole dissolves in water. By filtration of the 4'-amino-2',6'-di-pyrazolyl-pyridine compound can be separated without any unreacted impurities.

In the following the synthesis of 4'-iodo-2',6'-di-pyrazolyl-pyridine (III) will be described.

Synthesis of 4'-iodo-2',6'-di-pyrazolyl-pyridine from 4'-amino-2',6'-di-pyrazolyl-pyridine requires the following chemicals: 4'-amino-2',6'-di-pyrazolyl-pyridine, KI, I₂ Isoamylnitrite of formula (CH₃)₂CH(CH₂)₂-O-N=O, Na₂S₂O₃, and MgSO₄ as dehydrating agent.

4'amino-2',6'-di-pyrazolyl-pyridine was taken in a mixed solvent of dichloromethane / isoamyl nitrit (1:1 ratio) and deaerated with nitrogen gas completely. Here isoamyl nitrate acts as a mild diazotization agent. To this mixture KI and I₂ were also added to generate potassium triodide.

Here KI₃ acts as an iodination agent. The whole reaction mixture was heated at 70 °C under nitrogen / argon atmosphere with constant stirring for 4 hours. The obtained dark solution was poured into a phase transfer solution of dichloromethane and saturated aqueous solution of Na₂S₂O₃ (300 ml : 300 ml each) and stirred vigorously. Here the aqueous Na₂S₂O₃ solution was used to remove the unreacted excess of iodine by forming water soluble compounds:

I₂ + 2 Na₂S₂O₃ → 2 NaI + NaS₄O₆

The formed orange coloured organic layer containes the desired 4'-iodo-2',6'-di-pyrazolyl-pyridine. The orange coloured di-chlormethane fraction was separated in a separating funnel and dried using anhydrous MgSO₄ and filtered. The dichloromethane solution was concentrated to a small volume and chramotographed on silica using n-hexane : dichloromethane (2 : 3) as an eluent. The first colourless fraction was collected and the solvent was removed on a rotary evaporator to get a white coloured powder of 4'-iodo-2',6'-di-pyrazolyl-pyridine in a very high yield (≥ 95 %).

The following scheme shows the preparation of further compounds with 4'-iodo-2',6'-di-pyrazolyl-pyridine as an educt.

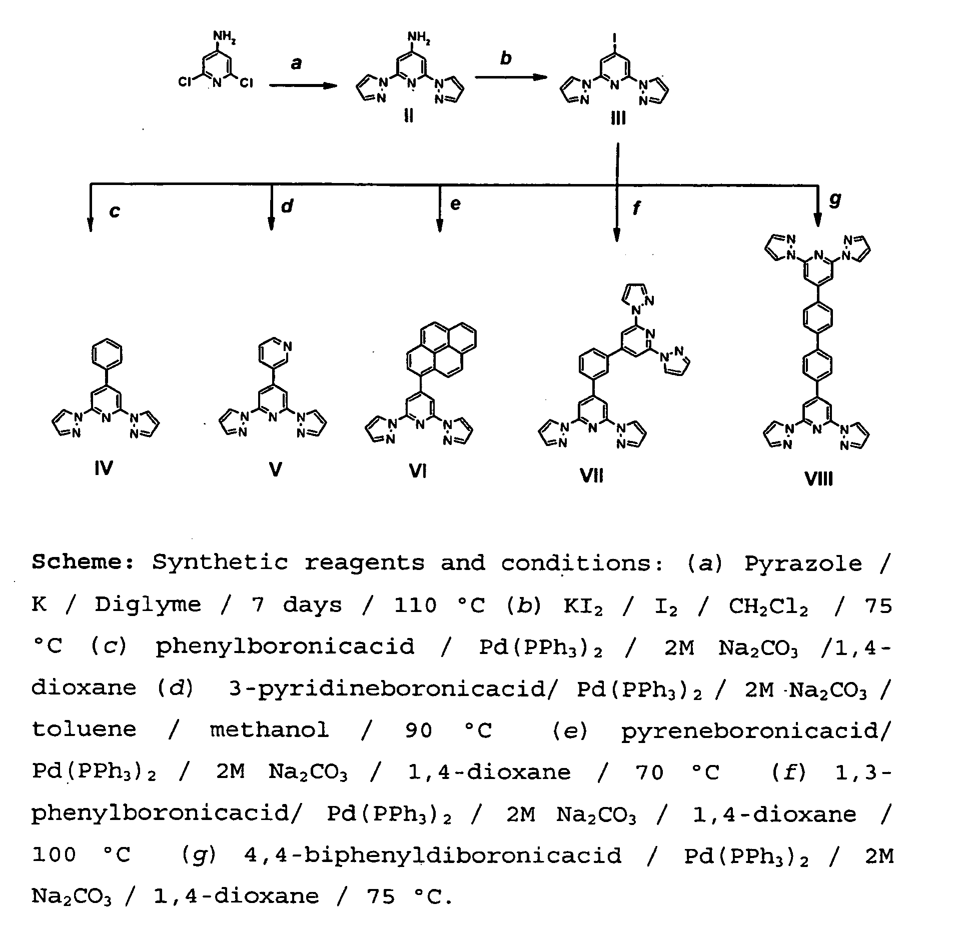

The invention will be described in detail by the following examples:
Preparation of 4'-amino-2',6'-bispyrazolylpyridine (II):
   In a clean and dry 250 mL two-neck flask, small pieces of K metal flakes (3.48 g, 88.78 mmol) were dispersed completely in diglyme (200 mL) by vigorous stirring under N₂ atmosphere. To this pyrazole (6.12g, 90.0 mmol) was added slowly to minimize the violent reaction that occurred due to the formation of potassium salt of pyrazole. The mixture was stirred for about 30 minutes at 70 °C until all the K was reacted to pyrazole to afford white turbid solution. 4-amino-2,6-dichloropyridine (4.9 g, 30 mmol) was added into this reaction mixture and heated to reflux at 110 °C for 7 days. The mixture was poured into water and the precipitate that formed was filtered and dried to isolate 4.9 g of compound II (72% yield). 1H NMR (300 MHz, CDCl3, 25 °C): δ = 8.55 (dd, 2H, 3JH,H = 2.64 Hz; 4JH,H = 0.75 Hz), 7.73 (dd, 2H, 3JH,H = 2.45 Hz; 4JH,H = 0.76 Hz), 7.14 (s, 2H, pyridine), 6.48 (m, 2H, pyrazole), 4.53 (s, 2H, -NH2) ppm. 13C NMR (75 MHz, DMSO-d6, 25 °C): δ = 159.7, 151.3, 142.6, 128.4, 108.3, 94.2 ppm. FT-IR (KBr) : ν = 3358 (ms, -NH₂ stretching), 3245 (ms, -NH₂ stretching), 1663 (s, -NH₂ scissor-ing) cm-1. MALDI-TOF m/z (relative intensity of isotopic distribution in %): 226.86 (100%), 227.86 (30%). C₁₁ H₁₀ N₆ (226.8): Calcd. C 58.40, H 4.46, N 37.15; found C 58.09, H 4.13, N 36.99.
Preparation of 4'-iodo-2',6'-bispyrazolylpyridine (III): 2,6-Di-pyrazol-1-yl-pyridin-4-ylamine (2.0 g, 8.3 mmol), iodine (4g) and KI (4g) were suspended in a mixed solvents of dichloromethane/i-amyl nitrite (1:1, 40 mL). The mixture was heated with stirring for 5 hrs. The mixture was poured into a saturated aqueous solution of Na2S2O3 (300 mL) and extracted with dichloromethane (3 times with 200 mL solution). The collected organic layers (orange coloured) were dried over MgSO4 and the solvent was evaporated. The crude orange product was purified by column chromatography on silica using a mixture of n-Hexane:CH₂Cl₂ (2:3) solvents as an eluent, the collected colourless fractions were combined and after solvent evaporation afforded white colour product III (2.78 g, yield >99%). 1H NMR (300 MHz, CDCl₃, 25 °C): δ = 8.51 (dd, 2H, 3JH,H = 4.15 Hz; 4JH,H = 1.51 Hz), 8.26 (s, 2H, pyridine), 7.77 (dd, 2H, 3JH,H = 3.2 Hz; 4JH,H = 0.56 Hz), 6.48 (m, 2H, 3JH,H = 2.07 Hz; 4JH,H = 0.56 Hz, pyrazole) ppm. 13C NMR (75 MHz, CDCl3, 25 °C): δ = 150, 143.1, 127.46, 124.49, 118.72, 109.1, 108.7 ppm. MALDI-TOF m/z (relative intensity of isotopic distribution in %): 337.62 (100%), 338.62 (25%).
Preparation of 4'-phenyl-2',6'-bispyrazolylpyridine (IV):
   4'-iodo-2',6'-dipyrazolyl-pyridine (0.337g, 1 mmol), phenylboronicacid (0.122 g, 1 mmol) and Pd(PPh₃)₄ (0.200 g, 0.173 mmol) were suspended in a N₂ gas bubbled solution of 1, 4-dioxane (20 mL) and 2M Na2CO3 (5 mL) and heated to 70°C for 3 days under nitrogen atmosphere. The 1,4-dioxane was removed using a rotary evaporator and the remaining residue was treated with water and extracted with CH₂Cl₂ solvent. The separated organic layer was dried over MgSO₄ and the solvent was removed by evaporation. The solid yellow residue was washed with methanol to remove the soluble impurities and to afford a white coloured powder. The white powder was washed through a short silica bed using dichloromethane and the resultant solution upon evaporation yielded bright white powder of IV (0.100 g, yield 35%). 1H NMR (300 MHz, CDC13, 25 °C): δ = 8.63 (dd, 2H), 8.14 (s, 2H), 7.81 (m, 4H), 7.51 (d, 2H), 6.53 (m, 2H) ppm. 13C NMR (75 MHz, CDC13, 25 °C): 154.2, 150.5, 142.3, 137.4, 129.7, 129.0, 127.2, 107.9, 107.3 ppm.
Preparation of V:
   4'-iodo-2',6'-dipyrazolyl-pyridine (1.348g, 4 mmol), 3-pyridineboronicacid (0.492 g, 4 mmol) and Pd(PPh₃)₄ (0.231g, 0.2 mmol, 10%) were suspended in a N₂ gas bubbled solution of toluene/methanol (20ml:20ml) and 2M Na₂CO₃ (5 mL) and heated to 90°C for 3 days under nitrogen atmosphere. The mixtures of solvents were removed by using a rotary evaporator and the remaining residue was treated with water and extracted with CH₂Cl₂ solvent. The separated organic layer was dried over MgSO₄ and the solvent was removed by evaporation. The solid residue was column chromatographed on silica at first with dichloromethane eluent to remove the coloured impurities and then with ethylacetate to get colourless fractions. The combined colourless solutions upon evaporation yielded white powder of V (1.1g, yield 94%). 1H NMR (300 MHz, CDC13, 25 °C): δ = 9.07 (d, 1H), 8.73 (s, 1H), 8.62 (d, 2H), 8.12 (d, 2H), 8.09 (m, 1H), 7.08 (s, 2H), 7.45 (q, 1H), 6.54 (m, 2H) ppm. 13C NMR (75 MHz, CDCl3, 25 °C): 151, 150.8, 150.7, 148.2, 142.5, 134.5, 133.3, 127.2, 123.7, 108.1, 107.2 ppm.
Preparation of VI:
   4'-iodo-2',6'-dipyrazolyl-pyridine (0.674g, 2 mmol), pyreneboronicacid (0.492 g, 2 mmol) and Pd(PPh₃)₄ (0.231 g, 0.2 mmol, 10%) were suspended in a N₂ gas bubbled solution of 1, 4-dioxane (150 mL) and 2M Na₂CO₃ (5 mL) and heated to 75°C for 3 days under nitrogen atmosphere. The 1,4-dioxane was removed using a rotary evaporator and the remaining residue was treated with water and extracted with CH₂Cl₂ solvent. The separated organic layer was dried over MgSO₄ and the solvent was removed by evaporation. The solid yellow residue was washed with methanol to remove the soluble impurities and to afford a white coloured powder. The solid residue was column chromatographed on silica with dichloromethane eluent and the second colourless fraction was collected and the resultant combined solution upon evaporation yielded bluish white powder of VI (0.270 g, yield 33%). 1H NMR (300 MHz, CDCl3, 25 °C): δ = 8.73 (dd, 2H), {8.28, 8.27, 8.25, 8.26, 8.24, 8.23, 8.22 (m, 4H pyrene)}, 8.18 (s, 2H), {8.17, 8.14, 8.13, 8.11, 8.09, 8.08, 8.07, 8.05, 8.02 (m, 5H, pyrene)}, 7.78 (d, 2H), 6.55 (m, 2H) ppm.
Preparation of VII:
   4'-iodo-2',6'-dipyrazolyl-pyridine (1.5 g, 4.45 mmol) and 1,3-phenyldiboronicacid (0.37 g, 2.2 mmol), and Pd(PPh₃)₄ (0.200 g, 0.173 mmol) were suspended in a N₂ gas bubbled solution of 1, 4-dioxane (150 mL) and 2M Na₂CO₃ (10 mL) and heated to 100°C for 3 days under nitrogen atmosphere. The 1,4-dioxane was removed using a rotary evaporator and the remaining residue was treated with water and extracted with CH₂Cl₂ solvent. The separated organic layer was dried over MgSO₄ and the solvent was removed by evaporation. The solid residue was column chromatographed on silica with dichloromethane eluent to remove the soluble yellow colour impurities followed by washing with ethylacetate to get a colourless liquid fraction. The colourless fraction upon evaporated on a rotor afforded compound VII as white powder (0.550 g, yield 25%). 1H NMR (300 MHz, CDC13, 25 °C): δ = 8.65 (dd, 4H), 8.3 (s, 1H), 8.20 (s, 4H), 7.94 (dd, 2H), 7.81 (s, 4H), 7.68 (t, 1H), 6.55 (m, 4H) ppm. 13C NMR (75 MHz, CDCl3, 25 °C): 153.6, 150.8, 142.6, 138.6, 130.0, 128.7, 127.4, 126.1, 108.2, 107.5 ppm. MALDI-TOF m/z (relative intensity of isotopic distribution in %): Observed: 496.77 (100%), 497.77 (40%), 498.77 (5%). Simulated: 496.19 (100%), 497.19 (30%), 498.19 (5%).
Preparation of VIII:
   4'-iodo-2',6'-dipyrazolyl-pyridine (0.674 g, 2 mmol) and 4,4-biphenyldiboronicacid (0.242 g, 1 mmol), and Pd(PPh₃)₄ (0.200 g, 0.173 mmol) were suspended in a N₂ gas bubbled solution of 1, 4-dioxane (50 mL) and 2M Na₂CO₃ (5 mL) and heated to 70°C for 5 days under nitrogen atmosphere. The 1,4-dioxane was removed using a rotary evaporator and the remaining residue was treated with water and extracted with CHCl₃ solvent immediately for several times. The separated organic layer was dried over MgSO₄ and the solvent was removed by evaporation. The solid residue was with dichloromethane and methanol to remove the soluble impurities to afford a white colour powder of compound VIII (0.480 g, yield 42%). 1H NMR (300 MHz, CDCl₃, 25 °C): δ = 8.65 (dd, 4H), 8.2 (s, 4H), 7.98 (s, 2H), 7.96 (s, 2H), 7.82 (m, 8H), 6.55 (m, 4H) ppm. 13C NMR (75 MHz, CDCl3, 25 °C): 150.7, 142.4, 141.5, 136.8, 127.8, 127.3, 108.1, 107.1 ppm. MALDI-TOF m/z (relative intensity of isotopic distribution in %): Observed: 572.82 (100%), 573.82 (45%), 574.82 (8%). Simulated: 572.21 (100%), 573.21 (40%), 574.21 (7.5%).

## Claims

1. Preparation of 2',6'-di-pyrazolyl-pyridine containing a reactive amino group in 4' position by reaction of
- 2,6-di-chloro-pyridine containing said reactive amino group in 4 position with
- a pyrazolate anion.

2. Preparation according to claim 1, where said reactive amino group R of 2',6'-di-pyrazolyl-pyridine is exchanged with a second organic or inorganic rest R².

3. Preparation according to claim 2, where rest R² is phenyl (IV), pyridine (V), pyrene(VI), 3-phenyl-(2',6'-bispyrazolylpyridine) (VII) or biphenyl-(2',6'bispyrazolylpyridine) (VIII).

## Patentansprüche

1. Verfahren zur Herstellung von 2',6'-Dipyrazolylpyridine, die eine reaktive Aminogruppe in 4'-Position enthält, durch eine Reaktion von
- 2,6-Dichlorpyridine, das die genannte reaktive Aminogruppe in 4'-Position enthält
mit
- einem Pyrazolatanion.

2. Verfahren nach Anspruch 1, wobei die reaktive Aminogruppe R in 2',6'-Dipyrazolylpyridine gegen einen zweiten organischen oder anorganischen Rest R² ausgetauscht wird.

3. Verfahren nach Anspruch 2, wobei der Rest R² Phenyl (IV), Pyridine (V), Pyrene(VI), 3-Phenyl-(2',6'-Bispyrazolyl-pyridine) (VII) oder Biphenyl-(2',6'-Bispyrazolyl-pyridine) (VIII) ist.

## Revendications

1. Préparation de 2',6'-di-pyrazolyl-pyridine renfermant un groupe amino réactif en position 4' par réaction de :
- 2,6-di-chloro-pyridine renfermant ce groupe amino réactif en position 4, avec
- un anion pyrazolate.

2. Préparation conforme à la revendication 1,
selon laquelle
le groupe amino réactif R de la 2',6'-di-pyrazolyl-pyridine est échangé avec un second groupe R² organique ou inorganique.

3. Préparation conforme à la revendication 2,
selon laquelle
le groupe R² est un groupe phényl (IV), pyridine (V), pyrène (VI), 3-phényl-(2',6'-bispyrazolylpyridine) (VII) ou biphényl-(2',6'-bispyrazolylpyridine) (VIII).
